# EUROPEAN PATENT APPLICATION

(11) **EP 2 172 559 A1**
(43) Date of publication of application: **07.04.2010**
(21) Application number: 08777159.8
(22) Date of filing: 12.06.2008
(51) Int. Cl.: C12Q 1/02, A61P 13/08, A61P 35/00, G01N 33/15, G01N 33/50, A61K 31/40, A61K 31/4155, A61K 31/427, A61K 31/4439, A61K 45/00, C07D 207/34, C07D 401/06, C07D 403/06, C07D 417/06

(54) **SCREENING METHOD**

(30) Priority: 13.06.2007 JP 2007156245
(71) Applicant: Takeda Pharmaceutical Company Limited, Osaka 541-0045 (JP)
(72) Inventor: HARA, Takahito, Tsukuba-shi Ibaraki 300-4293 (JP); YAMAOKA, Masuo, Tsukuba-shi Ibaraki 300-4293 (JP)
(74) Representative: Böhm, Brigitte
(86) International application number: PCT/JP2008/060743
(87) International publication number: WO 2008/153091

(57) **Abstract**

It is intended to provide a screening method of a compound having an effect of inhibiting the production of a prostatic specific antigen and/or an effect of suppressing the proliferation of androgen-independent prostatic cancer, comprising using an androgen-independent human prostatic cancer cell.

## Description

### Technical Field

The present invention relates to a screening method of a compound having an effect of inhibiting the production of prostatic specific antigens and/or an effect of suppressing the proliferation of androgen-independent prostatic cancer, wherein the method is characterized by using androgen-independent human prostatic cancer cells.

### Background Art

Androgen is a so-called male hormone that is synthesized in the testis and adrenal cortex, binds to an androgen receptor in a target organ, and shows various physiological activities. Naturally occurring androgens all chemically belong to C19 steroids. Among them, the major androgen is testosterone mainly synthesized in a testis, which shows enhanced uptake into a target cell and strong biological activity. In females, adrenal cortex is a major source of androgen.

Androgen is involved, for example, in the development and functional maintenance of the genital organs (including prostatic, seminal vesicle, epididymis and seminal duct), sex differentiation in embryonic stage, spermatogenesis, the development of secondary sexual characteristics (including induction of masculinization such as muscle-skeleton, voice, and fat distribution), the promotion of protein anabolism in muscle or the like, and bone metabolism.

When androgen is involved in pathological progression, an androgen reducing therapy is employed. For example, in androgen-dependent prostatic cancer, the therapeutic effects are achieved by reducing testosterone and its active metabolite, dihydrotestosterone or antagonizing their actions by castration (e.g., surgical castration and medical castration) or hormone therapies such as administration of an androgen-receptor antagonist.

Prostatic cancer that has reacquired the ability to proliferate after once the proliferation is suppressed by an androgen ablation therapy is called androgen-independent prostatic cancer.

When a patient with androgen-dependent prostatic cancer is treated, for example, by a hormone therapy, the blood level of prostatic specific antigen (PSA), a tumor marker, in the patient is reduced. However, if the hormone therapy is continued, after a certain period of time, persistent elevation of the blood level of PSA which once has been reduced is observed. This type of prostatic cancer which is unresponsive to the hormone therapy is also called androgen-independent prostatic cancer.

Conventional androgen-receptor antagonists act as an antagonist in model cells for androgen-dependent prostatic cancer and as an agonist or have no effect in model cells for androgen-independent prostatic cancer. If a compound can be found that acts as an antagonist in both the model cells for androgen-dependent prostatic cancer and androgen-independent prostatic cancer, such a compound is a potential therapeutic agent for androgen-independent prostatic cancer.

Although screening methods for androgen antagonists are disclosed in Patent Documents 1 to 4 and Non-Patent Documents 1 to 12, none of these are screen methods for finding a compound that acts as an antagonist in both the model cells for androgen-dependent prostatic cancer and androgen-independent prostatic cancer.

LNCap-FGC and LNCap-hr are known as established human prostatic cancer cell lines (Non-Patent Document 13).
Patent Document 1: WO03/057669
Patent Document 2: WO2004/016576
Patent Document 3: WO2006/064944
Patent Document 4: WO2007/015567
Non-Patent Document 1: Journal of Medicinal Chemistry, 1973, 16(5), 512-516
Non-Patent Document 2: J. Med. Chem., 1988, 31, 954-959
Non-Patent Document 3: J. Med. Chem., 1991, 34, 447-455
Non-Patent Document 4: J. Med. Chem., 1998, 41, 623-639
Non-Patent Document 5: Bioorganic & Medicinal Chemistry, 10, (2002), 1555-
Non-Patent Document 6: Eur. J. Med. Chem., 37, (2002), 619-634
Non-Patent Document 7: Bioorganic & Medicinal Chemistry Letters, 15, (2005), 271-276
Non-Patent Document 8: Bioorganic & Medicinal Chemistry Letters, 15, (2005), 389-393
Non-Patent Document 9: Chem. Pharm. Bull., 53(4), 402-409, (2005)
Non-Patent Document 10: Bioorganic & Medicinal Chemistry, 13, (2005), 2837-2846
Non-Patent Document 11: Bioorganic & Medicinal Chemistry Letters, 13, (2005), 6414-6424
Non-Patent Document 12: J. Med. Chem., 2006, 49, 716-726
Non-Patent Document 13: J. ancer Res. 2003 Jan. 1; 63(1): 149-53

### Disclosure of Invention

### Problems to Be Solved by the Invention

A screening method of a compound having an effect of inhibiting the production of prostatic specific antigens of androgen-independent prostatic cancer and/or an effect of suppressing the proliferation of the androgen-independent prostatic cancer has been desired.

### Means for Solving the Problems

The present inventors have conducted intensive research and found that a screening of a compound having an effect of inhibiting a production of prostatic specific antigens of an androgen-independent prostatic cancer and/or an effect of suppressing a proliferation of the androgen-independent prostatic cancer can be easily conducted by using androgen-independent human prostatic cancer cells. Based on this finding, the present invention has been completed.

More specifically, the present invention relates to:
(1) A screening method of a compound having an effect of inhibiting the production of a prostatic specific antigen and/or an effect of suppressing the proliferation of androgen-independent prostatic cancer, comprising using an androgen-independent human prostatic cancer cell.
(2) The screening method of (1), comprising a step of measuring and comparing a prostatic specific antigen production and cell numbers of androgen-independent human prostatic cancer cells which are contacted with a test compound and a prostatic specific antigen production and cell numbers of androgen-independent human prostatic cancer cells which are not contacted with the test compound.
(3) The screening method of (1), comprising the step of measuring and comparing an expression level of a reporter gene in an androgen-independent human prostatic cancer cell transfected with a reporter gene linked to a PSA promoter contacted with the test compound and that which is not contacted with the test compound.
(4) The screening method of (1), wherein the androgen-independent human prostatic cancer cell the androgen-independent human prostatic cancer cell is derived from LNCaP-FGC.
(5) The screening method of (1), wherein the androgen-independent human prostatic cancer cell is LNCaP-hr.
(6) The screening method of (1), which is a screening method of an antitumor agent.
(7) A screening method of a compound having an antitumor effect, comprising using an animal model transplanted with an androgen-independent human prostatic cancer cell.
(8) The screening method of (7), comprising a step of transplanting the androgen-independent human prostatic cancer cell into the model animal, and measuring and comparing state of cell proliferation in the model animal with and without administration of a test compound.
(9) The screening method of (7), wherein the androgen-independent human prostatic cancer cell is derived from LNCaP-FGC.
(10) The screening method of (7), wherein the androgen-independent human prostatic cancer cell is LNCaP-hr.
(11) The screening method of (7), wherein the animal model is a mouse or rat.
(12) A kit for screening a compound having an effect of inhibiting the production of a prostatic specific antigen and/or an effect of suppressing the proliferation of androgen-independent prostatic cancer, wherein the kit comprises an androgen-independent human prostatic cancer cell.
(13) A kit for screening a compound having an antitumor effect, wherein the kit comprises an androgen-independent human prostatic cancer cell.
(14) The screening kit of (12) or (13), wherein the androgen-independent human prostatic cancer cell is LNCaP-hr.

### Effects of the Invention

The screening method of the present invention allows a convenient screening of a compound having an effect of inhibiting the production of prostatic specific antigens of androgen-independent prostatic cancer and/or an effect of suppressing the proliferation of the androgen-independent prostatic cancer.

### Brief Description of Drawings

Fig. 1a is a graph showing change in a cell number when Compound 1 described in Example 1 is used. In the graph, the vertical axis represents the cell number (cells/well) and the horizontal axis represents the concentration of the compound (µM).
Fig. 1b is a graph showing change in a cell number when Compound 2 described in Example 1 is used. In the graph, the vertical axis represents the cell number (cells/well) and the horizontal axis represents the concentration of the compound (µM).
Fig. 1c is a graph showing change in a cell number when Compound 3 described in Example 1 is used. In the graph, the vertical axis represents the cell number (cells/well) and the horizontal axis represents the concentration of the compound (µM).
Fig. 1d is a graph showing change in a cell number when Compound 4 described in Example 1 is used. In the graph, the vertical axis represents the cell number (cells/well) and the horizontal axis represents the concentration of the compound (µM).
Fig. 1e is a graph showing change in a cell number when Compound 5 described in Example 1 is used. In the graph, the vertical axis represents the cell number (cells/well) and the horizontal axis represents the concentration of the compound (µM).
Fig. 1f is a graph showing change in a cell number when Compound 6 described in Example 1 is used. In the graph, the vertical axis represents the cell number (cells/well) and the horizontal axis represents the concentration of the compound (µM).
Fig. 1g is a graph showing change in a cell number when Compound 7 described in Example 1 is used. In the graph, the vertical axis represents the cell number (cells/well) and the horizontal axis represents the concentration of the compound (µM).
Fig. 1h is a graph showing change in a cell number when Compound 8 described in Example 1 is used. In the graph, the vertical axis represents the cell number (cells/well) and the horizontal axis represents the concentration of the compound (µM).
Fig. 1i is a graph showing change in a cell number when Compound 9 described in Example 1 is used. In the graph, the vertical axis represents the cell number (cells/well) and the horizontal axis represents the concentration of the compound (µM).
Fig. 2a is a graph showing the production of PSA when Compound 1 described in Example 1 is used. In the graph, the vertical axis represents the production of PSA (ng/mL) and the horizontal axis represents the concentration of the compound.
Fig. 2b is a graph showing the production of PSA when Compound 2 described in Example 1 is used. In the graph, the vertical axis represents the production of PSA (ng/mL) and the horizontal axis represents the concentration of the compound.
Fig. 2c is a graph showing the production of PSA when Compound 3 described in Example 1 is used. In the graph, the vertical axis represents the production of PSA (ng/mL) and the horizontal axis represents the concentration of the compound.
Fig. 2d is a graph showing the production of PSA when Compound 4 described in Example 1 is used. In the graph, the vertical axis represents the production of PSA (ng/mL) and the horizontal axis represents the concentration of the compound.
Fig. 2e is a graph showing the production of PSA when Compound 5 described in Example 1 is used. In the graph, the vertical axis represents the production of PSA (ng/mL) and the horizontal axis represents the concentration of the compound.
Fig. 2f is a graph showing the production of PSA when Compound 6 described in Example 1 is used. In the graph, the vertical axis represents the production of PSA (ng/mL) and the horizontal axis represents the concentration of the compound.
Fig. 2g is a graph showing the production of PSA when Compound 7 described in Example 1 is used. In the graph, the vertical axis represents the production of PSA (ng/mL) and the horizontal axis represents the concentration of the compound.
Fig. 2h is a graph showing the production of PSA when Compound 8 described in Example 1 is used. In the graph, the vertical axis represents the production of PSA (ng/mL) and the horizontal axis represents the concentration of the compound.
Fig. 2i is a graph showing the production of PSA when Compound 9 described in Example 1 is used. In the graph, the vertical axis represents the production of PSA (ng/mL) and the horizontal axis represents the concentration of the compound.
Fig. 3a is a graph showing reporter activity when Compound 6 described in Example 3 is used. In the graph, the vertical axis represents the reporter activity (%) and the horizontal axis represents the concentration of the compound.
Fig. 3b is a graph showing reporter activity when Compound 8 described in Example 3 is used. In the graph, the vertical axis represents the reporter activity (%) and the horizontal axis represents the concentration of the compound.
Fig. 3c is a graph showing reporter activity when Compound 9 described in Example 3 is used. In the graph, the vertical axis represents the reporter activity (%) and the horizontal axis represents the concentration of the compound.
Fig. 3d is a graph showing reporter activity when bicalutamide is used as a negative control compound. In the graph, the vertical axis represents the reporter activity (%) and the horizontal axis represents the concentration of the compound.

### Best Mode for Carrying Out the Invention

The screening method of the present invention for a compound having an effect of inhibiting the production of prostatic specific antigens of androgen-independent prostatic cancer and/or an effect of suppressing the proliferation of the androgen-independent prostatic cancer is characterized by using androgen-independent human prostatic cancer cells.

As used herein, the term "androgen-independent human prostatic cancer cells" refers to human prostatic cancer cells that show proliferative capacity under the culture conditions substantially in the absence of steroids (for example, molecules that bind to a wild-type or mutated androgen receptor; more specifically, for example, androgen, progesterone, glucocorticoid, and estrogen).

The androgen-independent human prostatic cancer cells include, for example: (i) human prostatic cancer cells that are derived from human prostatic cancer cells (parent cell line) of which proliferation is suppressed under the culture conditions substantially in the absence of steroids, and that show higher proliferative capacity than that of the parent cell line under the culture conditions substantially in the absence of steroids; (ii) human prostatic cancer cells that are derived from patients with prostatic cancer of which blood PSA level was raised again after it had once been reduced by a hormone therapy; and the like. The parent cell line described in (i) includes human prostatic cancer cells of which proliferation rate is reduced under the culture conditions substantially in the absence of steroids, and cells that are unable to proliferate under those conditions.

The preferred androgen-independent human prostatic cancer cells include, for example, in addition to the property described in (i), (iii) cells that show the increased basal production of PSA as compared to the parent cell line; and/or (iv) cells that show PSA production comparable to that of the parent cell line by androgen stimulation at a lower concentration than in the parent cell line. The cells of (iii) include those showing at least 1.2-fold, preferably 1.2-fold to 30-fold higher basal production of PSA than that in the parent cell line. The cells of (iv) include those showing PSA production comparable to that in the parent cell line by androgen stimulation at a concentration of one-third or less, preferably one-third to one-thirtieth the concentration used for the parent cell line.

The cells of (i) may be obtained, for example, by culturing, under the culture conditions substantially in the absence of steroids, human prostatic cancer cells (parent cell line) of which proliferation is suppressed under the culture conditions substantially in the absence of steroids, and then selecting a subline that has acquired higher proliferative capacity than that of the parent cell line. An example of the human prostatic cancer cells that can be used as the parent cell line includes LNCaP-FGC (ATCC CRL-1740).

The androgen-independent human prostatic cancer cells of the present invention include, for example, cells derived from LNCaP-FGC; more specifically, the human prostatic cancer cell LNCaP-hr described in Reference Example. The cells derived from LNCaP-FGC may be obtained, for example, by using the following procedure: First, culturing the parent cell line, LNCaP-FGC cell in a medium (for example, MEM medium, DMEM medium, RPMI1640 medium, 199 medium, and F12 medium; particularly RPMI1640 medium) supplemented with serum from which steroids have been removed by charcoal-dextran treatment; then, continuing to culture the cells under the same conditions after the cells start to proliferate again after the reduction of cell proliferation; and thereby obtaining cells with stable properties such as growth rate, PSA production, and cell morphology.

In the LNCaP-hr cells, the basal production of PSA, an androgen-responsive protein, is increased as compared with that in the parent cell line (LNCaP-FGC cells). Also, the LNCaP-hr cells show the PSA production comparable to that in the parent cell line by androgen stimulation at a lower concentration than in the parent cell line. Thus, the LNCaP-hr cells are considered to have higher responsiveness to androgen stimulation than the parent cell line. In the LNCaP-hr cells, the conventional androgen-receptor antagonist, bicalutamide acts as an agonist for the PSA production at higher concentration and the suppression of cell proliferation by bicalutamide is far lower than that in LNCap-FGC. These properties resemble those of prostatic cancers that are resistant to a hormone therapy in clinical practice.

The screening method of the present invention is characterized by comprising a step of:
(1) measuring and comparing the PSA production or cell numbers of androgen-independent human prostatic cancer cells which are contacted with a test compound and the PSA production or cell numbers of those which are not contacted with the test compound;
(2) contacting androgen-independent human prostatic cancer cells transfected with a reporter gene linked to a PSA promoter with a test compound; and
(3) transplanting androgen-independent human prostatic cancer cells into model animals, and measuring and comparing the state of cell proliferation in the animals with and without administration of a test compound.

The above steps (1) to (3) are detailed below.

(1) The screening method comprising the step of measuring and comparing the PSA production or cell numbers of androgen-independent human prostatic cancer cells which are contacted with a test compound and the PSA production or cell numbers of those which are not contacted with the test compound:
The method described in (1) may be used as a screening method for a compound (for example, a antitumor agent) having an effect of inhibiting the production of prostatic specific antigens and/or an effect of suppressing the proliferation of androgen-independent prostatic cancer, the method being characterized by using androgen-independent human prostatic cancer cells. For example, cells derived from LNCaP-FGC, particularly such as LNCaP-hr, may be used as the androgen-independent human prostatic cancer cells.

The measurement of the production of PSA may be conducted by culturing the androgen-independent prostatic cancer cells in a medium containing (or not containing) a test compound, followed by measuring PSA secreted into the culture medium using ELISA, RIA, and the like. The cell numbers can be determined after culturing the androgen-independent prostatic cancer cells in the medium containing (or not containing) a test compound. The cell numbers can be counted by a method such as cell counting using a Coulter counter, quantification of the cellular ATP content, and a method using a dye that develops a color when it is reduced by mitochondria.

Based on the results obtained in the step (1), a compound that is expected to have an effect of inhibiting the production of prostatic specific antigens and/or an effect of suppressing the proliferation of androgen-independent prostatic cancer may be obtained by: (i) selecting a test compound that reduces the PSA production of the androgen-independent human prostatic cancer cells when it is contacted with the cells; or (ii) selecting a test compound that suppresses the proliferation of the androgen-independent human prostatic cancer cells when it is contacted with the cells.

The screening method described in (1) may be carried out as exemplified below.

Androgen-independent human prostatic cancer cells derived from LNCaP-FGC are plated on multiwell plates or culture dishes, and a compound is added thereto on the next day. The compound is dissolved in an appropriate solvent (PBS, HBSS, saline, water, ethanol, DMSO, DMF, etc.) that dissolves the compound, and they are added to the multiwell plates or culture dishes plated with the cells, either directly or after additional dilution with a medium. After culturing the cells for a certain period of time, the concentration of PSA in the culture medium is determined by ELISA or RIA; then the suppression rate of PSA production is calculated based on the assumption that the rate for the control group is 100% and the PSA level before the addition of the compound is 0%. Cell proliferation is assessed by cell counting with a Coulter counter, determination of the cellular ATP content, BrdU uptake, [³H]-thymidine uptake, and a method using a dye (such as MTT, alamar blue, resazurin, WST-1, WST-8, etc.).

(2) The screening method comprising the step of contacting androgen-independent human prostatic cancer cells transfected with a reporter gene linked to a PSA promoter with a test compound:
The method described in (2) preferably comprises the step of measuring and comparing an expression level of a reporter gene in an androgen-independent human prostatic cancer cell transfected with a reporter gene linked to a PSA promoter contacted with the test compound and that which is not contacted with the test compound.

The method described in (2) may be used as a screening method for a compound (for example, a antitumor agent) having an effect of inhibiting the production of prostatic specific antigens and/or an effect of suppressing the proliferation of androgen-independent prostatic cancer, the method being characterized by using androgen-independent human prostatic cancer cells. For example, cells derived from LNCaP-FGC, particularly such as LNCaP-hr, may be used as the androgen-independent human prostatic cancer cells.

The term "PSA promoter" refers to a promoter of a PSA gene, the transcription of which is controlled by an androgen receptor. Available PSA promoters include 6k-PSA promoter (Document name: Mol. Endocrinol., 1997, 11, 148-161), PSAR-PCPSA-P (Document name: Cancer Res., 1997, 57, 495-499) and the like. For example, beta-galactosidase gene, luciferase gene, fluorescent protein gene, alkaline phosphatase gene, horseradish peroxidase gene, and chloramphenicol acetyltransferase gene may be used as the reporter genes.

The method described in (2) may be carried out as exemplified below. First, a PSA promoter is linked to a reporter gene and introduced into androgen-independent human prostatic cancer cells to generate a reporter cell line, and then the reporter cell line is contacted with a test compound. A compound (for example, an antitumor agent) having an effect of inhibiting the production of prostatic specific antigens and/or an effect of suppressing the proliferation of androgen-independent prostatic cancer may be obtained by selecting a compound that reduces the expression of the reporter gene when the compound is contacted with the reporter cell line; that is, a compound that has an effect of suppressing the transcriptional activation by androgen receptors.

(3) The screening method comprising the step of transplanting androgen-independent human prostatic cancer cells into model animals, and measuring and comparing the state of cell proliferation in the animals with and without administration of a test compound:
The method described in (2) may be used as a screening method for a compound having antitumor activity. For example, cells derived from LNCaP-FGC, particularly such as LNCaP-hr, may be used as the androgen-independent human prostatic cancer cells. For example, rabbits, dogs, cats, guinea pigs, hamsters, mice, and rats; preferably, mice or rats may be used as a model animal. Preferably, the model animals are immunodeficient animals. The determination of the state of cell proliferation includes, for example, the volumetric measurement of a tumor size.

Based on the results obtained in the step (3), a compound that is expected to have antitumor activity may be obtained by selecting a compound capable of reducing tumor volume or suppressing the increase in tumor volume in the model animals.

The method described in (3) may be carried out as exemplified below.

Androgen-independent human prostatic cancer cells derived from LNCaP-FGC are suspended in PBS, HBSS, saline or Matrigel, and the cell suspension corresponding to 0.5 to 10 x 10⁶ cells is transplanted subcutaneously, intraperitoneally, intravenously, intraventricularly, intraosseously or intraprostatically in nude mice. Male nude mice or, in some cases, castrated male nude mice are used. When the tumor volume increases to a certain level (e.g., 100 mm³) or more, the animals are grouped based on the tumor volume and administered with the compound via oral route 1 to 4 times per day for 1 to 8 weeks. The tumor volume is calculated based on the assumption that the volume of the control group is 100% and the volume of the treated group at the start of the administration is 0% (T/C %).

The test compounds subjected to the screening method of the present invention include, but are not limited to, compound libraries prepared using combinatorial chemistry technology, random peptide libraries prepared using solid-phase synthesis or phage display, or natural components derived from microorganisms, animals and plants, marine organisms, etc.

The present invention also provides a kit suitable for carrying out the above screening methods. The kit for screening of the present invention comprises androgen-independent human prostatic cancer cells. As androgen-independent human prostatic cancer cells, those described above in the description of the screening method of the present invention may be used.

The kit for screening of the present invention may further comprise reagents for assaying PSA. For example, the kit may comprise the following reagents: Tandem-R free PSA, highly sensitive PSA tandem, Access Hybritech free PSA, ST E Test "TOSOH" II (PSA II), Nanopia PSA, LT auto Wako PSA, Ranream PSA II, Markit M PA, ARCHITECT^{R} (Registered Trademark) PSA, Innotech PSA, DPC Immulyze (R) HS PSA, E-Test PSA, ECLusys PSA II, ECLusys FPSA, AxSYM^{R} PSA Dainapack^{R}, AxSYM^{R} free PSA Dainapack^{R}, Lumipulse PSA-N, PSA assay reagents for Lumispot AL-1000, assay reagents for Unicel Dx I 800, assay reagents for the HITACHI 7070 autoanalyzer.

The present invention also provides a prophylactic or therapeutic agent against cancer (particularly prostatic cancer) comprising a compound having an effect of inhibiting the production of prostatic specific antigens and/or an effect of suppressing the proliferation of androgen-independent prostatic cancer, the compound being obtained by the screening method of the present invention. The prophylactic or therapeutic agent may comprise, if necessary, a pharmacologically acceptable carrier.

As used herein, the term "pharmacologically acceptable carriers" refers to various organic or inorganic carrier substances conventionally used as materials for formulations. The pharmacologically acceptable carrier is blended as an excipient, a lubricant, a binder and a disintegrant in a solid formulation; and as a solvent, a solubilizing agent, a suspending agent, an isotonicity agent, a buffer agent and a soothing agent in a liquid formulation. Other additives for formulations, such as preservatives, antioxidants, colorants and sweetening agents, may be used if necessary.

Preferred examples of the excipient include lactose, saccharose, D-mannitol, D-sorbitol, starch, pregelatinized starch, dextrin, crystalline cellulose, low-substituted hydroxypropyl cellulose, sodium carboxymethylcellulose, gum Arabic, dextrin, pullulan, light anhydrous silicic acid, synthetic aluminum silicate, magnesium aluminometasilicate and the like.

Preferred examples of the lubricant include magnesium stearate, calcium stearate, talc, colloidal silica and the like.

Preferred examples of the binder include pregelatinized starch, sucrose, gelatin, gum Arabic, methylcellulose, carboxymethylcellulose, sodium carboxymethylcellulose, crystalline cellulose, saccharose, D-mannitol, trehalose, dextrin, pullulan, hydroxypropyl cellulose, hydroxypropyl methylcellulose, polyvinylpyrrolidone and the like.

Preferred examples of the disintegrant include lactose, saccharose, starch, carboxymethylcellulose, calcium carboxymethylcellulose, croscarmellose sodium, sodium carboxymethyl starch, light anhydrous silicic acid, low-substituted hydroxypropyl cellulose and the like.

Preferred examples of the solvent include water for injection, saline, Ringer's solution, alcohol, propylene glycol, polyethylene glycol, sesame oil, corn oil, olive oil, cotton seed oil and the like.

Preferred examples of the solubilizing agent include polyethylene glycol, propylene glycol, D-mannitol, trehalose, benzyl benzoate, ethanol, tris-aminomethane, cholesterol, triethanolamine, sodium carbonate, sodium citrate, sodium salicylate, sodium acetate and the like.

Preferred examples of the suspending agent include surfactants such as stearyltriethanolamine, sodium lauryl sulfate, lauryl aminopropionic acid, lecithin, benzalkonium chloride, benzethonium chloride and glyceryl monostearate; hydrophilic polymers such as polyvinyl alcohol, polyvinylpyrrolidone, sodium carboxymethylcellulose, methylcellulose, hydroxymethyl cellulose, hydroxyethyl cellulose and hydroxypropyl cellulose; polysorbates, polyoxyethylene hydrogenated castor oil and the like.

Preferred examples of the isotonicity agent include sodium chloride, glycerin, D-mannitol, D-sorbitol, dextrose and the like.

Preferred examples of the buffer agent include phosphate, acetate, carbonate, citrate buffers and the like.

Preferred examples of the soothing agent include benzyl alcohol and the like.

Preferred examples of the preservative include paraoxybenzoate esters, chlorobutanol, benzyl alcohol, phenethyl alcohol, dehydroacetic acid, sorbic acid and the like.

Preferred examples of the antioxidant include sulfite, ascorbate and the like.

Preferred examples of the colorant include water-soluble edible tar dyes (for example, edible dyes such as Food Color Red Nos. 2 and 3, Food Color Yellow Nos. 4 and 5, and Food Color Blue Nos. 1 and 2), water-insoluble lake dyes (for example, aluminum salts of the above water-soluble edible tar colors), natural dyes (for example, β-carotene, chlorophyll and colcothar) and the like.

Preferred examples of the sweetening agent include saccharin sodium, dipotassium glycyrrhizinate, aspartame, stevia and the like.

The dosage form of the pharmaceutical composition includes, for example, oral formulations such as tablets, capsules (including soft capsules and microcapsules), granules, powders, syrups, emulsions and suspensions; and parenteral formulations such as injections (e.g., subcutaneous injections, intravenous injections, intramuscular injections, and intraperitoneal injections), external formulations (e.g., formulations for nasal administration, formulations for transdermal administration and ointments), suppositories (e.g., rectal suppositories and vaginal suppositories), pellet, drops, and sustained release formulations (e.g., sustained release microcapsules). These dosage forms can be safely administered either orally or parenterally.

The pharmaceutical composition may be prepared according to any method conventional in the field of pharmaceutical technology, for example, a method as described in the Japanese Pharmacopoeia. Specific methods for preparing formulations will be described in detail below. Although the content of the compound obtained by the screening method of the present invention in the pharmaceutical composition varies depending on the dosage form, the dose of the compound and the like, it is, for example, about 0.1% to 100% by weight.

For example, an oral formulation may be prepared by compression molding after adding an excipient (e.g., lactose, sucrose, starch, D-mannitol), a disintegrant (e.g., carboxymethylcellulose calcium), a binder (e.g., pregelatinized starch, gum Arabic, carboxymethylcellulose, hydroxypropyl cellulose, polyvinylpyrrolidone), or a lubricant (e.g., talc, magnesium stearate, polyethylene glycol 6000) to active ingredients, followed by, if necessary, coating the resulting product with a coating base for the purpose of taste masking, enteric dissolution or sustained release using a well-known method.

The coating base includes, for example, a sugar-coating base, a water-soluble film-coating base, an enteric film-coating base, a sustained release film-coating base and the like.

The sugar-coating base includes, for example, sucrose, which may be used in combination with one or more selected from talc, precipitated calcium carbonate, gelatin, gum Arabic, pullulan, Carnauba Wax, and the like.

The water-soluble film coating base includes, for example, cellulosic polymers such as hydroxypropyl cellulose, hydroxypropyl methylcellulose, hydroxyethyl cellulose and methylhydroxyethyl cellulose; synthetic polymers such as polyvinyl acetal diethylaminoacetate, aminoalkylmethacrylate copolymer E [Eudragit E (trade name), Roehm Pharma] and polyvinyl pyrrolidone; and polysaccharide such as pullulan.

The enteric film coating base includes, for example, cellulosic polymers such as hydroxypropyl methylcellulose phthalate, hydroxypropyl methylcellulose acetate succinate, carboxymethylethylcellulose and cellulose acetate phthalate; acrylic acid polymers such as (meth)acrylic acid copolymer L [Eudragit L (trade name), Roehm Pharma], (meth)acrylic acid copolymer LD [Eudragit L-30D55 (trade name), Roehm Pharma] and (meth)acrylic acid copolymer S [Eudragit S (trade name), Roehm Pharma]; and naturally occurring materials such as Shellac.

The sustained release film coating base includes, for example, cellulosic polymers such as ethyl cellulose; acrylic acid polymers such as aminoalkyl (meth)acrylate copolymer RS [Eudragit RS (trade name), Roehm Pharma] and ethyl acrylate-methyl (meth)acrylate copolymer suspension [Eudragit NE (trade name), Roehm Pharma].

Two or more of the above-described coating bases may be used in combination at an appropriate proportion. A light blocking agent such as titanium oxide or iron sesquioxide may also be used in coating.

The injection is prepared by dissolving, suspending or emulsifying the active ingredient together with additives such as a dispersant (e.g., Polysorbate 80, polyoxyethylene hydrogenated castor oil 60, polyethylene glycol, carboxymethyl cellulose and sodium alginate), a preservative (e.g., methyl parabene, propyl parabene, benzyl alcohol, chlorobutanol and phenol), and an tonicity agent (e.g., sodium chloride, glycerine, D-mannitol, D-sorbitol and glucose) in an aqueous solvent (e.g., distilled water, saline and Ringer's solution) or a lipid solvent (e.g., vegetable oils such as olive oil, sesame oil, cottonseed oil and corn oil and propylene glycol). In this process, other additives such as a solubilizing agent (e.g., sodium salicylate and sodium acetate), a stabilizer (e.g., human serum albumin) and a soothing agent (e.g., benzyl alcohol) may also be used. Generally, the injection is filled into appropriate ampules.

The formulations thus obtained are safe and low toxic, and can be administered, for example, to mammals (e.g., humans, mice, rats, rabbits, sheep, pigs, cattle, horses, cats, dogs, monkeys and chimpanzees) either orally or parenterally.

The dosage of the prophylactic or therapeutic agent varies depending on, for example, a disease to be treated, the severity of the disease, a subject to be treated, and an administration route, for example, for an adult patient with prostatic cancer (60 kg body weight), about 0.1 to about 100 mg, preferably about 1.0 to about 50 mg, more preferably about 1.0 to about 20 mg of the compound an active ingredient can be administered per day in a single dose or several divided doses.

The present invention is described further in detail below with reference to Reference Example and Examples, however, the present invention is not limited thereto.

### Examples

### Reference Example

The androgen-independent human prostatic cancer cells "LNCaP-hr" of the present invention were obtained using human prostatic cancer cells LNCaP-FGC as a parent cell line.

First, LNCaP-FGC cells (ATCC CRL-1740) were cultured using a medium substantially free of steroids. The medium substantially free of steroids was PRMI1640 medium (GIBCO, 11835-030, phenol red free) which contains 10% fetal bovine serum depleted of androgen.

The depletion of androgen from fetal bovine serum was carried out by treatment of the fetal bovine serum with DCC (Dextran coated charcoal). First, 25 g of "Charcoal, activated, USP" (SIGMA, C7606-500G), 250 mg of "Dextran T70" (EXTRASYNTHESE, 4445) and 500 mL of D-PBS (GIBCO: 14190-144) were mixed and then autoclaved (121°C, 20 minutes). This mixture was used as DCC. Then, 500 mL of fetal bovine serum was mixed with 25 mL of DCC and shaken and stirred in a warm water bath at 45°C for 30 minutes. Subsequently, the mixture was centrifuged twice at 3000 rpm at 4°C. The resulting supernatant was sterilized by filtration (CORNING 430773, 0.2 um Nylon), and fetal bovine serum depleted of androgen was obtained. The fetal bovine serum depleted of androgen was mixed with PRMI1640 medium (GIBCO, 11835-030, phenol red free) to obtain a medium substantially in the absence of steroids.

When cultured in the medium substantially free of steroids, the LNCaP-FGC cells started to proliferate again after their proliferation was suppressed once. After the restart of the proliferation, the culture was continued under the same culture conditions to select cells with stable growth rate and cell morphology. The cells thus obtained were designated "LNCaP-hr."

The cells "LNCaP-hr" are also found in Cancer Res. 2003 Jan. 1; 63(1): 149-53 (Non-Patent Document 13).

The basal PSA production of the LNCaP-hr cells was determined by ELISA. The results showed that the basal production of the androgen-responsive protein PSA in the LNCaP-hr cells was about 1.2 to about 30-fold higher than that of the LNCaP-FGC cells.

The PSA production of the cells stimulated with androgen was also determined by ELISA. The results showed that the PSA production of the LNCaP-hr cells was comparable to that of the LNCaP-FGC cells by androgen stimulation at a concentration as low as one-third to one-thirtieth of the concentration used for the LNCaP-FGC cells. Thus, the LNCaP-hr cells are considered to have higher responsiveness to androgen stimulation than the parent cell line.

In the NCaP-hr cells, the conventional AR antagonist bicalutamide acted as an agonist in the PSA production at higher concentration and the suppression of cell proliferation by bicalutamide was far lower than that in LNCap-FGC. These properties resembled those of prostatic cancers that are resistant to a hormone therapy in clinical practice.

Compounds were evaluated for the inhibition of PSA production and cell proliferation in vitro using the LNCaP-hr cells, and were evaluated for antitumor activity against the tumor generated by transplanting these cells into nude mice.

### Example 1

Test of inhibition of PSA production in LNCaP-hr cells by compounds:
Compounds 1-9 shown in Table 1 were used as test compounds to carry out the test of inhibition of PSA production. First, LNCaP-hr cells were plated on 24-well plates at a density of 40,000 cells/500 µL/well, to which the compounds were added on the next day. Three days after the addition, the concentration of PSA in the culture medium was determined by ELISA, and the rate of the inhibition of PSA production was calculated based on the assumption that the rate for the control group was 100% and the PSA level before the addition of the compounds was 0%. The cell numbers were also counted using a Coulter counter.

Compounds that reduced both the cell number and the PSA production, compared to those in the absence of the compounds were selected.

The compounds shown in Table 1 were prepared according to the description of the specifications of WO2006/046944 and Japanese Patent Application No. 2006-163992.

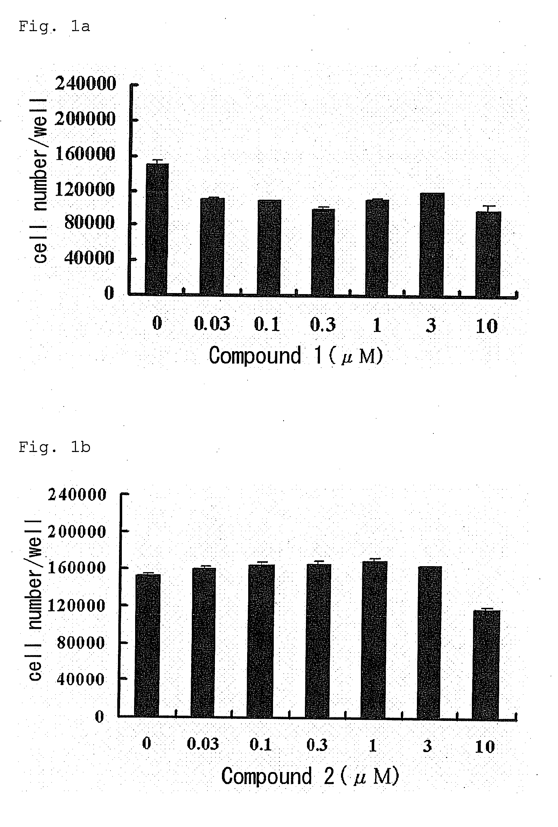

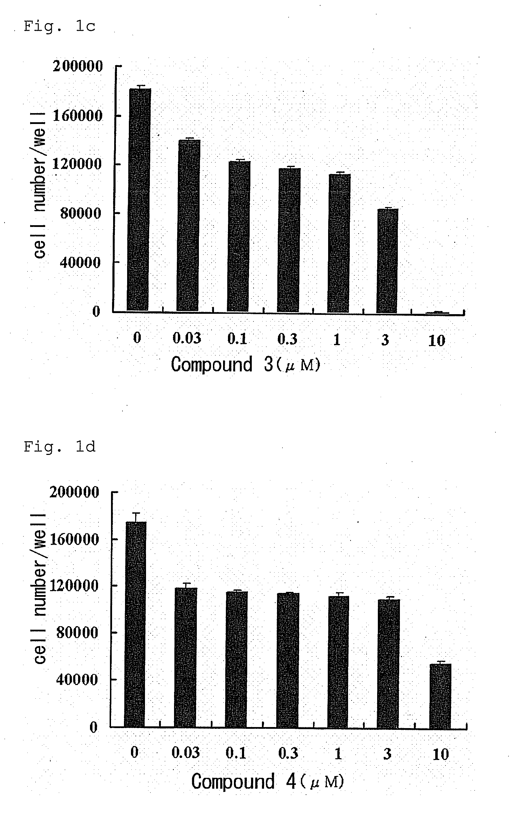

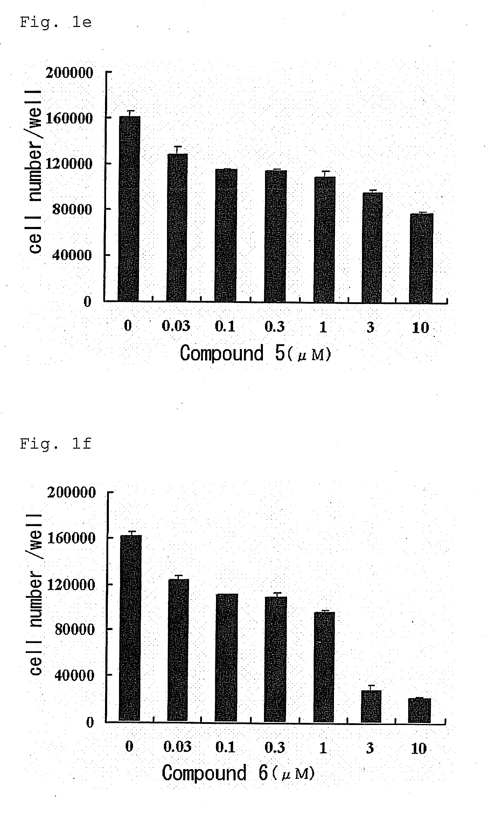

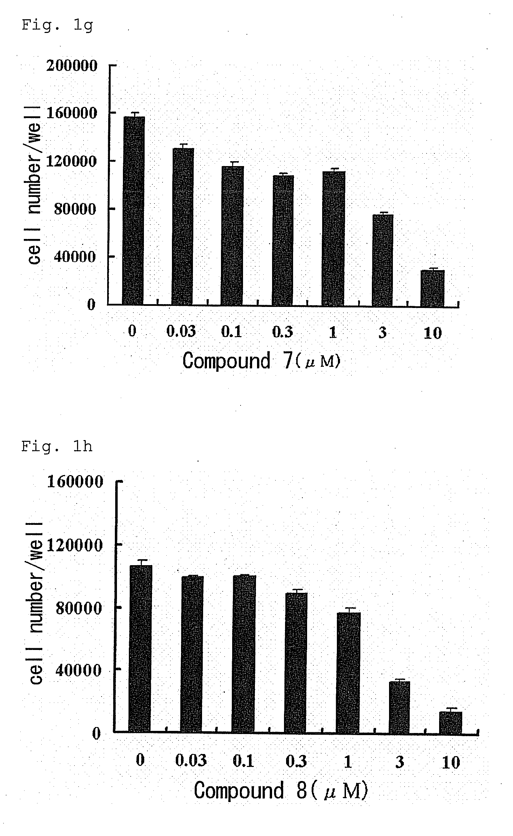

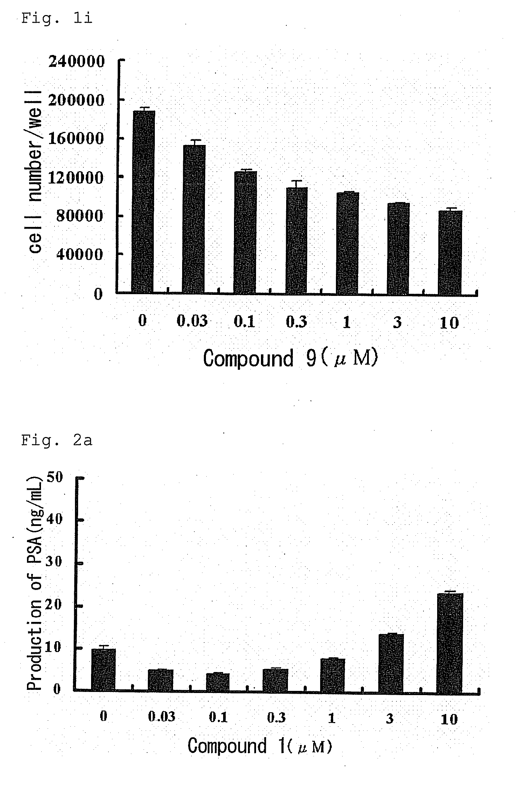

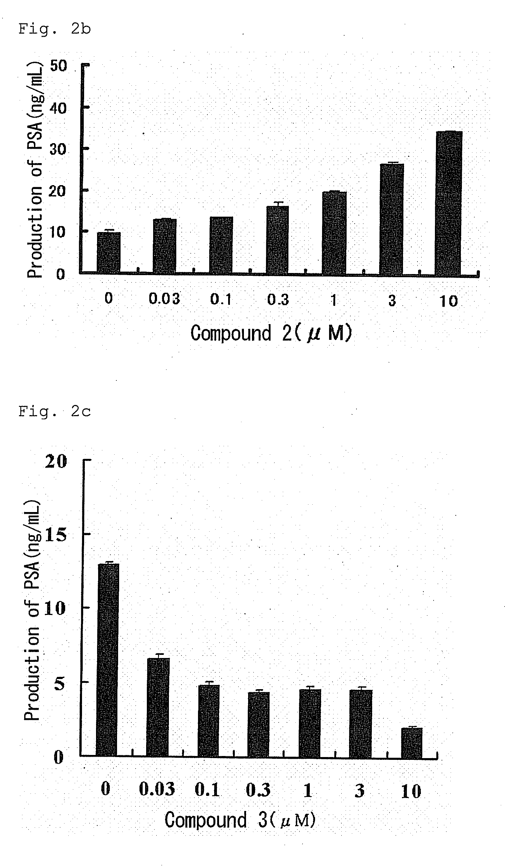

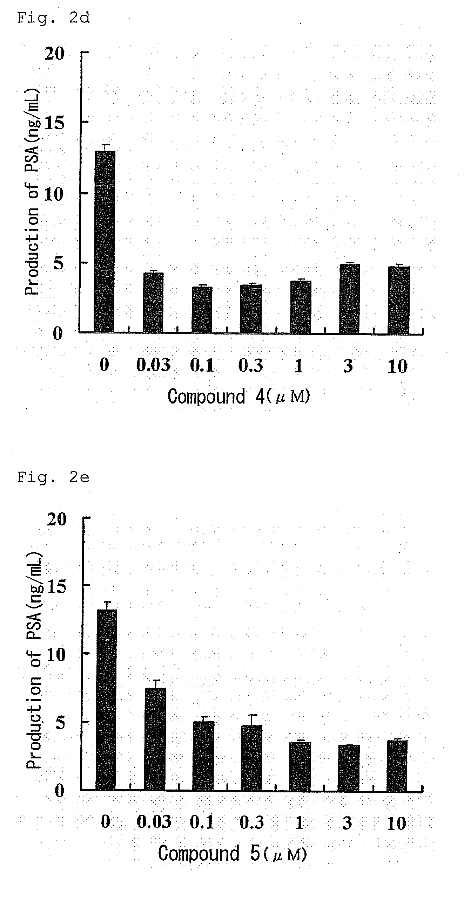

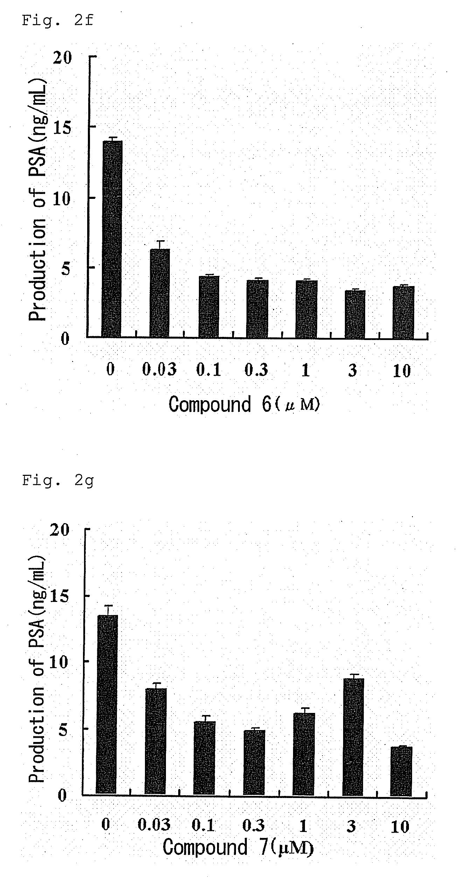

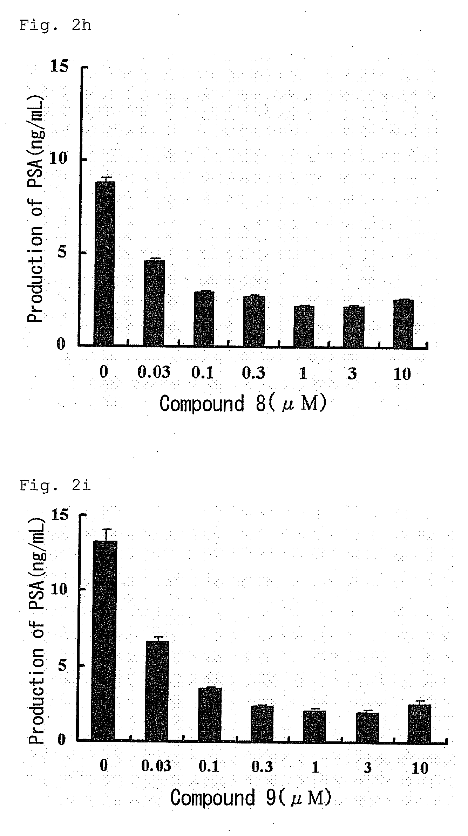

The results are shown in Figures 1 and 2. Effects of suppressing the production of PSA were observed for Compounds 3-9.

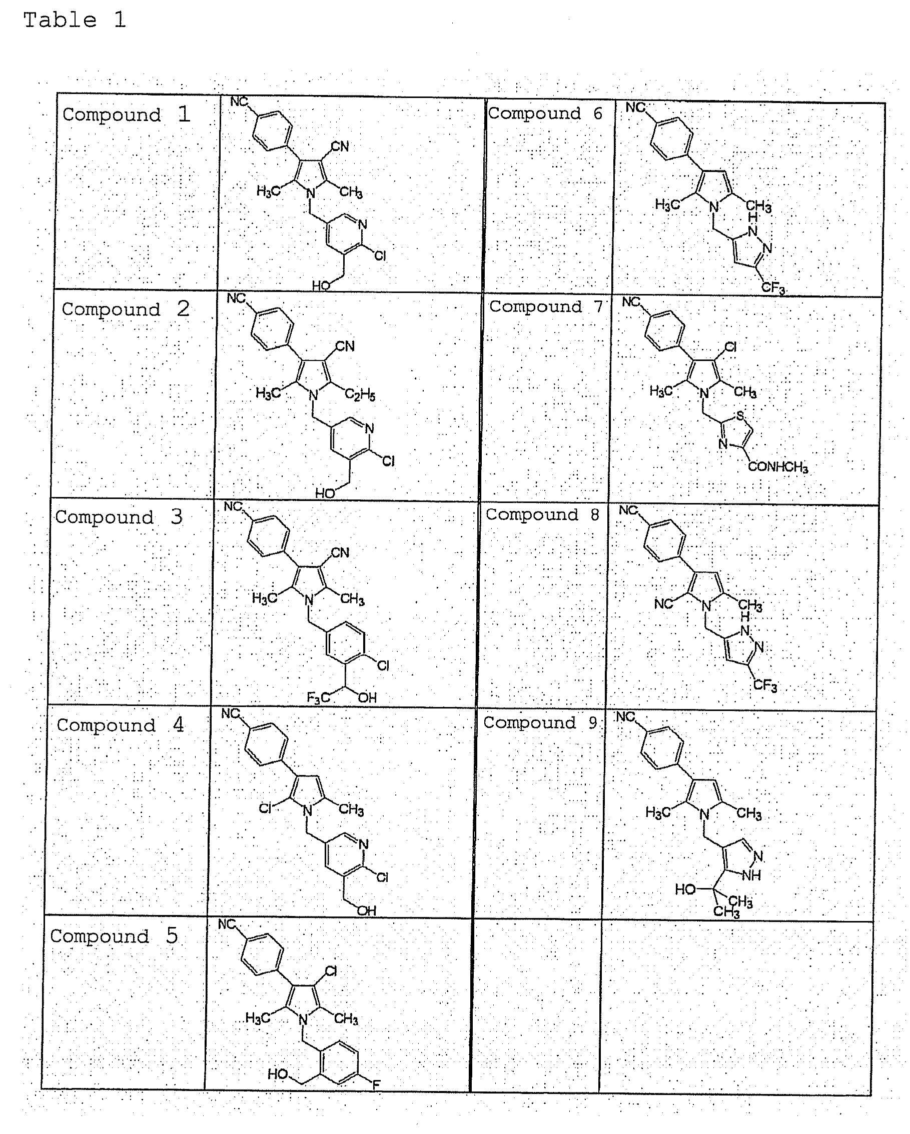

### Example 2

Antitumor test using nude mice transplanted with LNCaP-hr cells:
Antitumor tests were conducted using Compounds 1, 6 and 9 shown in Table 1 as test compounds. LNCaP-hr cells were suspended in Matrigel, and 5 x 10⁶ cells were transplanted subcutaneously into the flank of castrated nude mice. When tumor volume reached approximately 100 mm³, the animals were grouped based on the tumor volume, and administered orally with 15 mg/kg body weight of the compound twice daily for three weeks. The tumor volume was calculated based on the assumption that the volume of the control group was 100% and the volume of the treated group at the start of the administration was 0% (T/C %). The results are shown in Table 2. The antitumor activity was observed for all of Compounds 6, 8 and 9.

**Table 2**

| | |
|---|---|
| Compound 6 | T/C=-3% |
| Compound 8 | T/C=-5% |
| Compound 9 | T/C=24% |

### Example 3

Reporter assay using LNCaP-hr cells:
Reporter assay was performed using Compounds 6, 8 and 9 shown in Table 1 as test compounds. As a negative control compound, conventional AR antagonist, bicalutamide was used. 6k-PSA promoter and firefly luciferase gene were used as a PSA promoter and a reporter gene, respectively.

LNCaP-hr cells transfected with a reporter gene linked to a PSA promoter were plated on 96-well plates at a density of 50,000 cells/100 µL/well, to which the test compounds were added after the cells were cultured for 8 hours. The reporter activity was determined 24 hours after the addition of the test compounds. The suppressing activity of the test compounds on the reporter gene was calculated based on the assumption that the reporter activity in the cells without the addition of the test compounds (control group) was 100% and the reporter activity of the non-treated LNCaP-hr cells without introduction of the reporter gene was 0%.

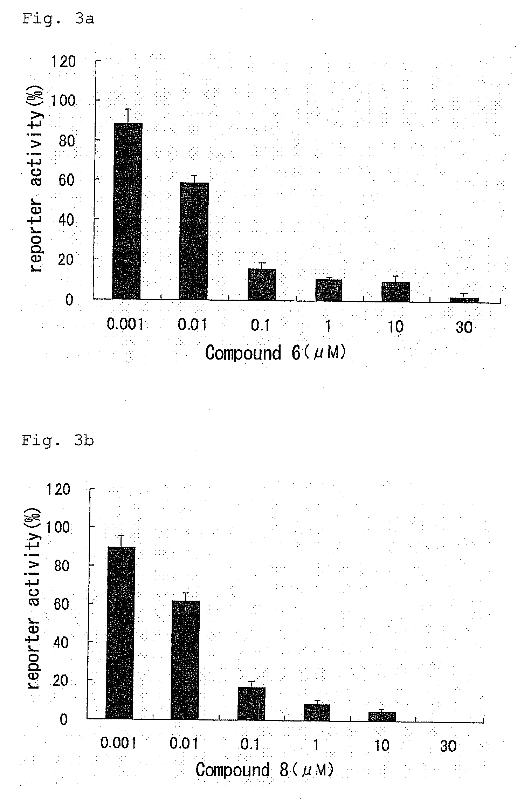

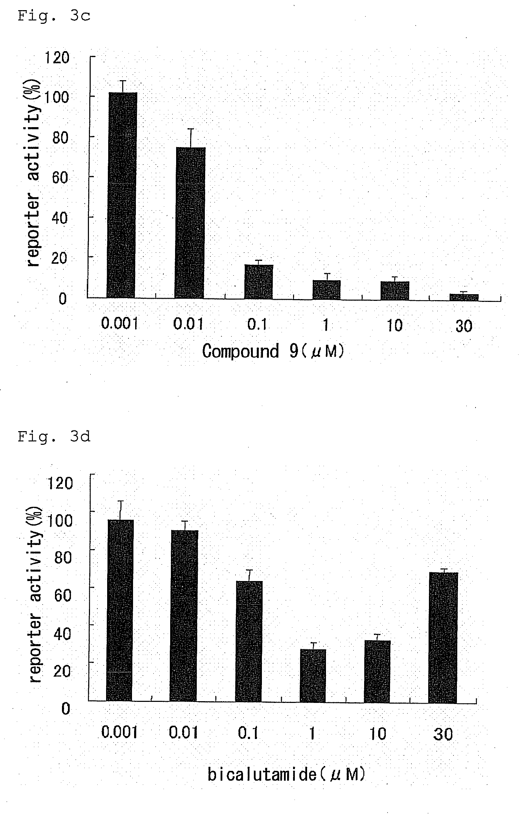

The results are shown in Figure 3. Concentration-dependent suppressing activity on the reporter gene was observed for all of Compounds 6, 8 and 9. Each of the test compounds was shown to have an effect of inhibiting the transcriptional activation by androgen receptors. On the other hand, the negative control bicalutamide did not show any concentration-dependent suppressing activity on the reporter gene.

### Industrial Applicability

The screening method of the present invention allows the convenient selection of a compound that has an effect of inhibiting the production of prostatic specific antigens and/or an effect of suppressing the proliferation of androgen-independent prostatic cancer.

## Claims

1. A screening method of a compound having an effect of inhibiting the production of a prostatic specific antigen and/or an effect of suppressing the proliferation of androgen-independent prostatic cancer, comprising using an androgen-independent human prostatic cancer cell.

2. The screening method according to claim 1, comprising a step of measuring and comparing a prostatic specific antigen production and cell numbers of androgen-independent human prostatic cancer cells which are contacted with a test compound and a prostatic specific antigen production and cell numbers of androgen-independent human prostatic cancer cells which are not contacted with the test compound.

3. The screening method according to claim 1, comprising the step of measuring and comparing an expression level of a reporter gene in an androgen-independent human prostatic cancer cell transfected with a reporter gene linked to a PSA promoter contacted with the test compound and that which is not contacted with the test compound.

4. The screening method according to claim 1, wherein the androgen-independent human prostatic cancer cell the androgen-independent human prostatic cancer cell is derived from LNCaP-FGC.

5. The screening method according to claim 1, wherein the androgen-independent human prostatic cancer cell is LNCaP-hr.

6. The screening method according to claim 1, which is a screening method of an antitumor agent.

7. A screening method of a compound having an antitumor effect, comprising using an animal model transplanted with an androgen-independent human prostatic cancer cell.

8. The screening method according to claim 7, comprising a step of transplanting the androgen-independent human prostatic cancer cell into the model animal, and measuring and comparing state of cell proliferation in the model animal with and without administration of a test compound.

9. The screening method according to claim 7, wherein the androgen-independent human prostatic cancer cell is derived from LNCaP-FGC.

10. The screening method according to claim 7, wherein the androgen-independent human prostatic cancer cell is LNCaP-hr.

11. The screening method according to claim 7, wherein the animal model is a mouse or rat.

12. A kit for screening a compound having an effect of inhibiting the production of a prostatic specific antigen and/or an effect of suppressing the proliferation of androgen-independent prostatic cancer, wherein the kit comprises an androgen-independent human prostatic cancer cell.

13. A kit for screening a compound having an antitumor effect, wherein the kit comprises an androgen-independent human prostatic cancer cell.

14. The screening kit according to claim 12 or 13, wherein the androgen-independent human prostatic cancer cell is LNCaP-hr.
